(19) Europäisches Patentamt
European Patent Office
Office européen des brevets

(11) Veröffentlichungsnummer: **0 345 597 B1**

(12) # EUROPÄISCHE PATENTSCHRIFT

(45) Veröffentlichungstag der Patentschrift: **27.10.93**

(51) Int. Cl.[5]: **C07F 9/40**, A01N 57/04, C07F 9/177, C07F 9/20, C07F 9/42, C07C 35/04

(21) Anmeldenummer: **89109686.9**

(22) Anmeldetag: **29.05.89**

(54) **O-Halogencyclobutyl-S-alkyl-(di)thiophosphon(r)säureester, Verfahren zu ihrer Herstellung und ihre Verwendung als Schädlingsbekämpfungsmittel.**

(30) Priorität: **09.06.88 DE 3819632**

(43) Veröffentlichungstag der Anmeldung: **13.12.89 Patentblatt 89/50**

(45) Bekanntmachung des Hinweises auf die Patenterteilung: **27.10.93 Patentblatt 93/43**

(84) Benannte Vertragsstaaten: **BE CH DE ES FR GB IT LI NL**

(56) Entgegenhaltungen:
**DE-A- 2 634 587**
**FR-A- 1 545 034**

**THE JOURNAL OF CHEMICAL PHYSICS, Band 25, Nr. 5, November 1956, Seiten 949-955; W.D. PHILIPS: "Fluorine magnetic resonance spectra of some mono- and disubstituted tetrafluorocyclobutanes"**

(73) Patentinhaber: **BAYER AG**

**D-51368 Leverkusen(DE)**

(72) Erfinder: **Krüger, Bernd-Wieland, Dr.**
**Unterboschbach 19**
**D-5060 Bergisch-Gladbach(DE)**
Erfinder: **Bielefeldt, Dietmar, Dr.**
**Beuthener Strasse 13**
**D-4030 Ratingen-Hösel(DE)**
Erfinder: **Gassen, Karl R., Dr.**
**Augenweg 6a**
**D-5068 Odenthal(DE)**
Erfinder: **Homeyer, Bernhard, Dr.**
**Obere Strasse 28**
**D-5090 Leverkusen 3(DE)**
Erfinder: **Becker, Benedikt, Dr.**
**Metzkausener Strasse 14**
**D-4020 Mettmann(DE)**
Erfinder: **Matthaei, Hans-Detlef, Dr.**
**Paul-Klee-Strasse 47**
**D-5090 Leverkusen 1(DE)**
Erfinder: **Stendel, Wilhelm, Dr.**
**In den Birken 55**
**D-5600 Wuppertal 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue O-Halogencyclobutyl-S-alkyl(di)thiophosphon(r)säureester, mehrere Verfahren zu ihrer Herstellung und ihre Verwendung als Schädlingsbekämpfungsmittel, insbesondere als Insektizide, Akarizide und Nematizide.

Es ist bereits bekannt, daß bestimmte O-(2,2,2-Trihalogenethyl)-S-(alkyl)-(di)thiophosphorsäureester, wie z.B. O-(Ethyl)-O-(2,2,2-trichlorethyl)-S-(n-propyl)-thiophosphorsäuresterund O-(Ethyl)-O-(2,2,2-trifluorethyl)-S-(n-propyl)-dithiophosphorsäureester zur Schädlingsbekämpfung verwendet werden können (vgl. DE-OS 2 732 930).

Die insektizide, akarizide und nematizide Wirkung der bekannten Verbindungen ist jedoch, insbesondere bei niedrigen Wirkstoffkonzentrationen und Aufwandmengen, nicht immer zufriedenstellend.

Es wurden nun neue O-Halogencyclobutyl-S-(alkyl)-(di) thiophosphon(r)-säureester der Formel (I)

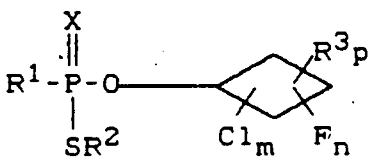

(I)

gefunden, in welcher

X für Sauerstoff oder Schwefel steht,

p für null, eins oder zwei steht,

m für null, eins oder zwei steht,

n für 2, 3 oder 4 steht,

$R^1$ für Alkyl, für gegebenenfalls durch Sauerstoff oder Schwefel unterbrochenes Alkoxy, welches durch Halogen substituiert sein kann, Alkenyloxy, Alkinyloxy, für Heterocyclyl-alkoxy oder für gegebenenfalls durch Alkyl und/oder Halogen substituiertes Cycloalkoxy steht,

$R^2$ für gegebenenfalls durch Sauerstoff oder Schwefel unterbrochenes Alkyl, welches durch Halogen substituiert sein kann, für Alkenyl, Alkinyl oder für Heterocyclyl-alkyl steht und

$R^3$ für Wasserstoff oder Alkyl steht.

Weiterhin wurde gefunden, daß man die neuen O-Halogencyclobutyl-S-alkyl(di)thiophosphor(n)-säureester der Formel (I) erhält,

wenn man

a) Thio- oder Dithiophosphor(n)säureester der Formel (II)

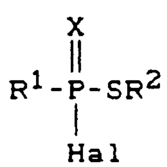

(II)

in welcher

$R^1$, $R^2$ und X die oben angegebene Bedeutung haben und

Hal für Halogen steht,

mit Cyclobutanol-Derivaten der Formel (III)

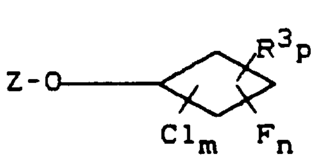

(III)

in welcher

$R^3$, n, m und p die oben angegebene Bedeutung haben und

Z für Wasserstoff oder ein Äquivalent eines Alkalimetallions steht, gegebenenfalls in Gegenwart von Verdünnungsmitteln und gegebenenfalls in Gegenwart von Basen umsetzt,

oder wenn man

b) zum Erhalt von Thio- oder Dithiophosphorsäureestern der Formel (I) in einem ersten Reaktionsschritt Säurehalogenide der Formel (IV)

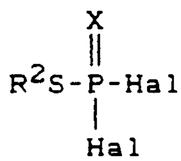

(IV)

in welcher

X, $R^2$ und Hal die oben angegebene Bedeutung besitzen,

mit Verbindungen der Formel

$R^4$-H (V)

in welcher

$R^4$ für die bei $R^1$ aufgeführten Reste, außer Alkyl, steht,

gegebenenfalls in Gegenwart von Lösungsmitteln und gegebenenfalls in Gegenwart von Basen umsetzt und dann in einem anschließenden Reaktionsschritt mit Cyclobutanol-Derivaten der Formel (III)

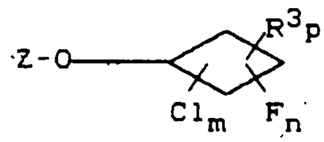

(III)

in welcher

Z, $R^3$, m, n und p die oben angegebene Bedeutung besitzen,

gegebenenfalls in Gegenwart von Verdünnungsmitteln und gegebenenfalls in Gegenwart von Basen umsetzt,

oder wenn man

c) zum Erhalt von Thio- oder Dithiophosphorsäureestern der Formel (I) in einem ersten Reaktionsschritt Säurehalogenide der Formel (VI)

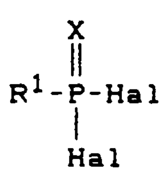

(VI)

in welcher

X, $R^1$ und Hal die oben angegebene Bedeutung haben,

mit Cyclobutanol-Derivaten der Formel (III)

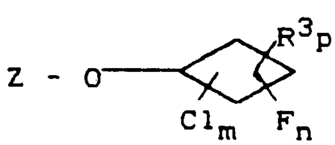

(III)

in welcher

Z, $R^3$, n, m und p die oben angegebene Bedeutung haben,

gegebenenfalls in Gegenwart von Verdünnungsmitteln und gegebenenfalls in Gegenwart von Basen umsetzt und in einem zweiten Reaktionsschritt die erhaltenen Verbindungen der Formel (VII)

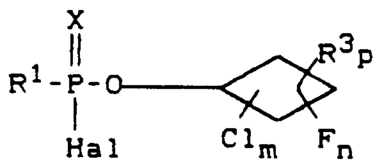

(VII)

in welcher

X, Hal, $R^1$, $R^3$, n, m und p die oben angegebene Bedeutung haben, mit Verbindungen der Formeln (VIIIa) bzw. (VIIIb)

$Z^1$-SH (VIIIa) bzw. $(Z^1)_2S$ (VIIIb)

in welcher

$Z^1$ für ein Äquivalent eines Alkalimetallions (vorzugsweise Natrium oder Kalium) steht,

gegebenenfalls in Gegenwart von Verdünnungsmitteln umsetzt und in einem dritten Reaktionsschritt die erhaltenen Verbindungen der Formel (IX)

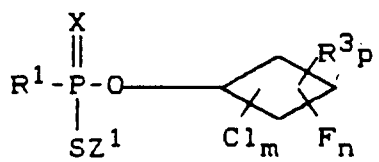

(IX)

in welcher

X, $Z^1$, $R^1$, $R^3$, m, n und p die oben angegebene Bedeutung haben, mit Alkylhalogeniden der Formel (X)

$R^2$ - Hal (X)

in welcher

$R^2$ und Hal die oben angegebene Bedeutung haben,

gegebenenfalls in Gegenwart von Verdünnungsmitteln umsetzt.

Schließlich wurde gefunden, daß die neuen O-Halogencyclobutyl-S-alkyl(di)thiophosphor(n)säureester der allgemeinen Formel (I) stark ausgeprägte insektizide, akarizide und nematizide Eigenschaften besitzen. Die neuen Verbindungen zeigen vor allem sehr starke nematizide Wirkungen und weisen eine günstige Verträglichkeit auf.

Die erfindungsgemäßen Verbindungen sind durch die Formel (I) allgemein definiert.

In den allgemeinen Formeln steht Alkyl für geradkettiges oder verzweigtes Alkyl mit vorzugsweise 1 bis 6, insbesondere 1 bis 4 Kohlenstoffatomen, wobei Methyl, Ethyl, n- und i- Propyl und n-, i-, s- und t-Butyl beispielhaft genannt seien.

Gegebenenfalls durch Sauerstoff oder Schwefel unterbrochenes Alkyl und Alkoxy bedeutet geradkettiges oder verzweigtes Alkoxyalkyl, Alkylthioalkyl, Alkoxyalkoxy und Alkylthioalkoxy mit vorzugsweise 1 bis 6, insbesondere 1 bis 4 Kohlenstoffatomen je Alkyl-, Alkoxy- oder Alkylthioteil.

Diese Reste können durch ein oder mehrere, gleich oder verschiedene Halogenatome (Fluor, Chlor, Brom und Jod, vorzugsweise Fluor, Chlor und Brom) substituiert sein.

Alkenyl, Alkinyl, Alkenyloxy und Alkinyloxy in den allgemeinen Formeln sind geradkettig oder verzweigt und enthalten vorzugsweise 2 bis 6, insbesondere 2 bis 4 Kohlenstoffatome und vorzugsweise 1 Doppel- oder Dreifachbindung.

Heterocyclyl-alkoxy und Heterocyclyl-alkyl sind geradkettig oder verzweigt und enthalten im Alkyl- oder Alkoxyteil vorzugsweise 1 bis 4, insbesondere 1 oder 2 Kohlenstoffatome. Der Heterocyclyl-Teil enthält

vorzugsweise 5 oder 6 Ringglieder und vorzugsweise 1 Heteroatom, wobei Sauerstoff als Heteroatom bevorzugt wird. Der heterocyclische Ring ist vorzugsweise gesättigt. Als Beispiele seien Tetrahydrofuranyl und Hexahydropyranyl als Heterocyclyl-Teile genannt.

Gegebenenfalls durch Alkyl und/oder Halogen substituiertes Cycloalkoxy enthält vorzugsweise 3 bis 6, insbesondere 4 bis 6 Ringglieder, wobei beispielhaft Cyclopropyloxy, Cyclobutyloxy, Cyclopentyloxy und Cyclohexyloxy genannt seien, Cycloalkyloxy kann 1 oder mehrfach, vorzugsweise 1 bis 5-fach durch gleiche oder verschiedene Substituenten substituiert sein. Alkyl als Substituent ist geradkettig oder verzweigt und enthält vorzugsweise 1 bis 4, insbesondere 1 oder 2 Kohlenstoffatome. Halogen als Substituent bedeutet Fluor, Chlor, Brom und Jod, vorzugsweise Fluor und Chlor, Beispielhaft sei der 3,3,2-Trifluor-2-chlor-cyclobutoxy-Rest genannt.

Hal bedeutet gleiches oder verschiedenes Halogen, vorzugsweise Chlor, Brom und Jod, insbesondere Chlor und Brom, besonders bevorzugt Chlor.

m steht vorzugsweise für 0 oder 1 und n steht vorzugsweise für 3 oder 4.

Der Rest

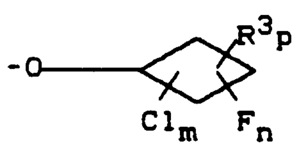

steht vorzugsweise für den Rest

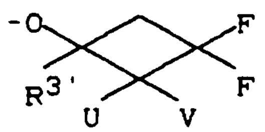 (IVa)

in welcher

- $R^{3'}$ für Wasserstoff oder $(C_1\text{-}C_4)$-Alkyl steht,
- U für Wasserstoff, Fluor oder Chlor steht und
- V für Wasserstoff, Fluor oder Chlor steht.

Bevorzugt steht $R^{3'}$ für Wasserstoff oder Methyl (vorzugsweise Wasserstoff), U für Fluor oder Chlor (vorzugsweise Chlor) und V für Fluor oder Chlor (vorzugsweise Fluor).

Bevorzugte erfindungsgemäße Verbindungen der Formel (I) sind die Verbindungen der allgemeinen Formel (Ia)

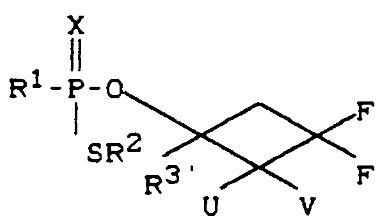 (Ia)

in welcher

- X für Sauerstoff oder Schwefel steht
- $R^{3'}$ für Wasserstoff oder $(C_1\text{-}C_4)$-Alkyl (vorzugsweise für Wasserstoff) steht und
- U für Wasserstoff, Fluor oder Chlor (vorzugsweise Fluor oder Chlor) steht,
- V für Wasserstoff, Fluor oder Chlor (vorzugsweise für Fluor) steht,
- $R^1$ für $(C_1\text{-}C_6)$-Alkyl, $(C_1\text{-}C_6)$-Alkoxy, $(C_2\text{-}C_6)$-Alkenyloxy, $(C_2\text{-}C_6)$-Alkinyloxy, (2-Hexahydropyranyl)-methoxy, (2-Tetrahydrofuranyl)-methoxy, $(C_1\text{-}C_6)$-Alkoxy-$(C_1\text{-}C_4)$-alkoxy, $(C_1\text{-}C_6)$-Alkylthio-$(C_1\text{-}C_4)$-alkoxy oder 3,3,2-Trifluor-2-chlor-cyclobutoxy (vorzugsweise $(C_1\text{-}C_6)$-Alkoxy) steht und
- $R^2$ für $(C_1\text{-}C_6)$-Alkyl, $(C_2\text{-}C_6)$-Alkenyl, $(C_2\text{-}C_6)$-Alkinyl, (2-Hexahydropyranyl)-methyl, (2-Tetrahydrofuranyl)-methyl, $(C_1\text{-}C_6)$-Alkylthio-$(C_1\text{-}C_4)$-alkyl oder $(C_1\text{-}C_6)$-Alkoxy-$(C_1\text{-}C_4)$-alkyl (vorzugsweise

($C_1$-$C_6$)-Alkyl) steht.

Besonders bevorzugt sind Verbindungen der Formel (Ia), in welcher

X für Sauerstoff oder Schwefel (vorzugsweise Sauerstoff) steht,

$R^{3'}$ für Wasserstoff oder Methyl (vorzugsweise für Wasserstoff) steht,

U für Wasserstoff, Fluor oder Chlor (vorzugsweise Fluor oder Chlor) steht,

V für Wasserstoff, Fluor oder Chlor (vorzugsweise Fluor) steht,

$R^1$ für ($C_1$-$C_4$)-Alkyl, ($C_1$-$C_4$)-Alkoxy, ($C_2$-$C_4$)-Alkenyloxy, ($C_2$-$C_4$)-Alkinyloxy, (2-Tetrahydropyranyl)-methoxy, ($C_1$-$C_4$)-Alkoxy-($C_1$-$C_4$)-alkoxy oder ($C_1$-$C_4$)-Alkylthio-($C_1$-$C_4$)alkoxy (vorzugsweise ($C_1$-$C_4$)-Alkoxy) steht und

$R^2$ für ($C_1$-$C_4$)-Alkyl, ($C_2$-$C_4$)-Alkenyl, ($C_2$-$C_4$)-Alkinyl, (2-Tetrahydrofuranyl)-methyl, ($C_1$-$C_4$)-Alkoxy-($C_1$-$C_4$)-alkyl oder ($C_1$-$C_4$)-Alkylthio-($C_1$-$C_4$)-alkyl (vorzugsweise ($C_1$-$C_4$)-Alkyl) steht.

Ganz besonders bevorzugt sind Verbindungen der Formel (Ia), in welcher

X für Sauerstoff oder Schwefel (vorzugsweise Sauerstoff) steht,

$R^{3'}$ für Wasserstoff oder Methyl (vorzugsweise Wasserstoff) steht,

U für Wasserstoff, Fluor oder Chlor (vorzugsweise Fluor oder Chlor) steht,

V für Fluor oder Chlor (vorzugsweise Fluor) steht,

$R^1$ für ($C_1$-$C_4$)-Alkyl, ($C_1$-$C_4$)-Alkoxy oder ($C_2$-$C_4$)-Alkenyloxy (vorzugsweise ($C_1$-$C_4$)-Alkoxy) steht und

$R^2$ für ($C_1$-$C_4$)-Alkyl oder ($C_2$-$C_4$)Alkenyl (vorzugsweise ($C_1$-$C_4$)-Alkyl) steht.

Im einzelnen seien außer den bei den Herstellungsbeispielen genannten Verbindungen die folgenden O-Halogencyclobutyl-S-alkyl-(di) thiophosphon(r)säureester der allgemeinen Formel (I) genannt:

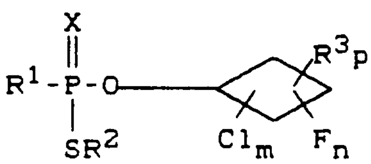

(I)

| $R^1$ | X | $R^2$ | 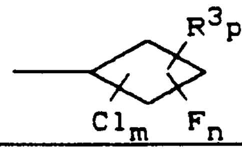 |
|---|---|---|---|
| $CH_3$ | S | $CH(CH_3)C_2H_5$ | 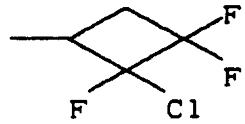 |
| $C_2H_5$ | S | $CH(CH_3)C_2H_5$ | 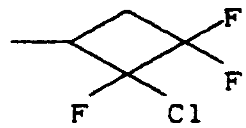 |
| $CH_3$ | S | $CH(CH_3)C_2H_5$ | 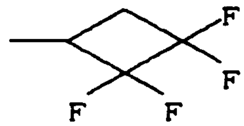 |
| $C_2H_5$ | S | $CH(CH_3)C_2H_5$ | 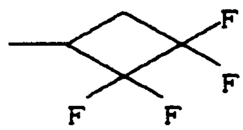 |
| $CH_3$ | S | $CH(CH_3)C_2H_5$ | 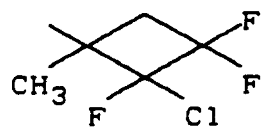 |
| $C_2H_5$ | S | $CH(CH_3)C_2H_5$ | 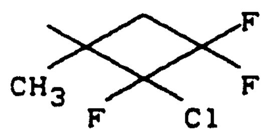 |
| $CH_3$ | S | $C_4H_9$-i | 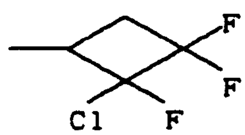 |

| $R^1$ | X | $R^2$ | 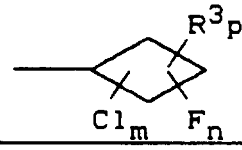 |
|---|---|---|---|
| $C_2H_5$ | S | $C_4H_9$-t | 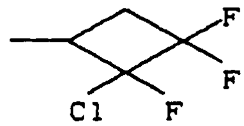 |
| $CH_3$ | O | $C_4H_9$-i | 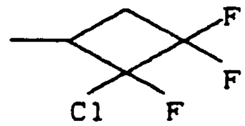 |
| $C_2H_5$ | O | $C_4H_9$-t | 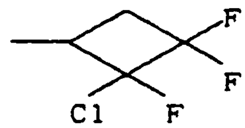 |
| $CH_3O$ | O | $C_3H_7$-n | 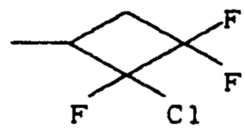 |
| $C_2H_5O$ | O | $C_3H_7$-n | 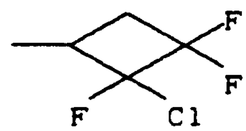 |
| $C_3H_7O$ | O | $C_3H_7$-n | 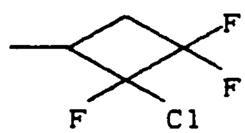 |
| $C_2H_5O$ | S | $C_3H_7$-n | 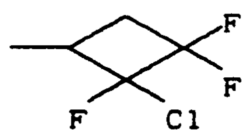 |
| $C_2H_5O$ | O | $C_3H_7$-n | 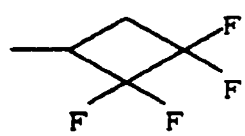 |

| $R^1$ | X | $R^2$ | 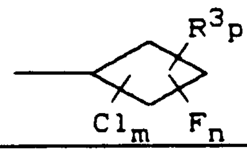 |
|---|---|---|---|
| $C_2H_5O$ | S | $C_3H_7$-n | 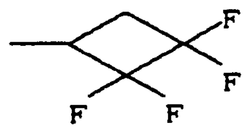 |
| $C_2H_5O$ | O | $C_3H_7$-n | 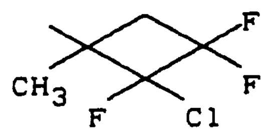 |
| $C_2H_5O$ | S | $C_3H_7$-n | 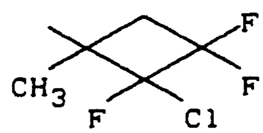 |
| $C_2H_5O$ | O | $CH(CH_3)C_2H_5$ | 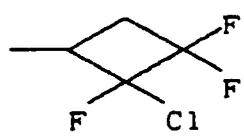 |
| $C_2H_5O$ | O | $CH(CH_3)_2$ | 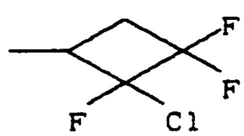 |
| $C_2H_5O$ | O | $C_4H_9$-t | 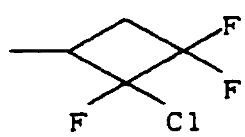 |
| $C_2H_5O$ | O | $CH(CH_3)C_2H_5$ | 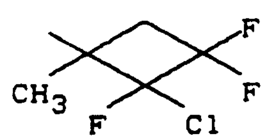 |
| $C_2H_5O$ | O | $CH(CH_3)_2$ | 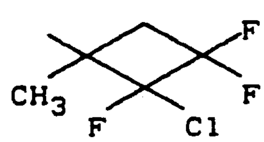 |

| $R^1$ | X | $R^2$ | 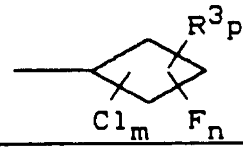 |
|---|---|---|---|
| $C_2H_5O$ | O | $C_4H_9$-t | 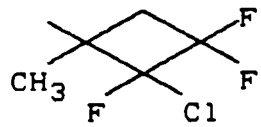 |
| $C_2H_5O$ | O | $CH(CH_3)C_2H_5$ | 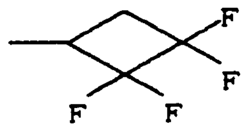 |
| $C_2H_5O$ | O | $CH(CH_3)_2$ | 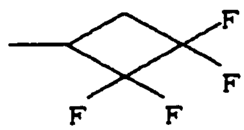 |
| $C_2H_5O$ | O | $C_4H_9$-t | 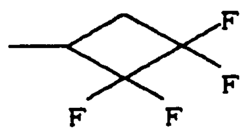 |
| $C_3H_7$ | O | $C_3H_7$ | 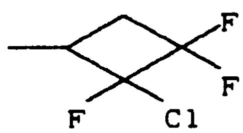 |
| $C_3H_7$ | S | $C_3H_7$ | 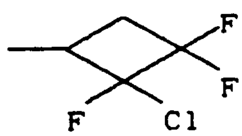 |
| $C_3H_7$ | O | $OH(CH_3)C_2H_5$ | 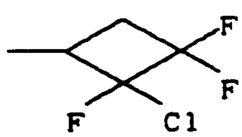 |
| $C_3H_7$ | S | $OH(CH_3)C_2H_5$ | 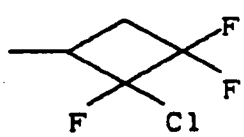 |
| $C_3H_7$-i | S | $C_3H_7$ | 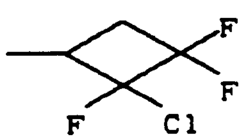 |

| $R^1$ | X | $R^2$ | 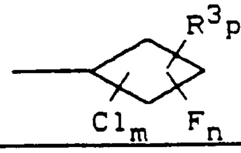 |
|---|---|---|---|
| $C_2H_5O$ | O | $C_4H_9$-i | 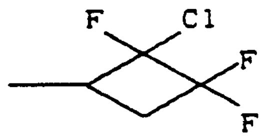 |
| $C_2H_5O$ | O | $C_4H_9$-i | 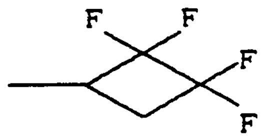 |
| $C_2H_5O$ | S | $C_4H_9$-i | 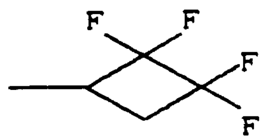 |
| $C_2H_5O$ | S | $C_4H_9$-i | 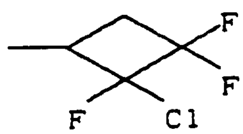 |
| $C_2H_5O$ | O | $CH(CH_3)CH=CH_2$ | 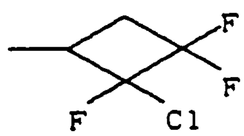 |
| $C_2H_5O$ | O | $CH(CH_3)CH=CH_2$ | 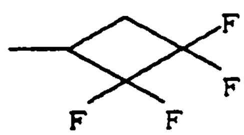 |
| $C_2H_5O$ | S | $CH(CH_3)CH=CH_2$ | 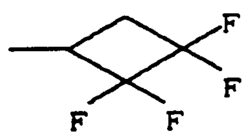 |
| $C_2H_5O$ | S | $CH(CH_3)CH=CH_2$ | 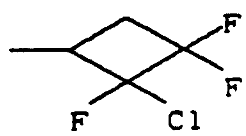 |

| $R^1$ | X | $R^2$ | 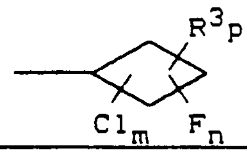 |
|---|---|---|---|
| $C_2H_5O$ | O | $CH_2CH{=}CH_2$ | 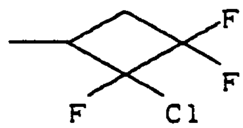 |
| $C_2H_5O$ | O | $CH_2CH{=}CH_2$ | 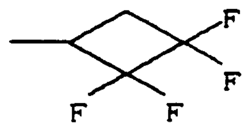 |
| $C_2H_5O$ | S | $CH_2CH{=}CH_2$ | 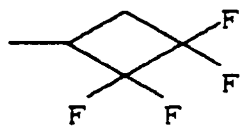 |
| $C_2H_5O$ | S | $CH_2CH{=}CH_2$ | 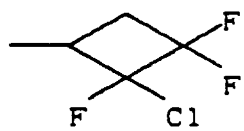 |
| $C_2H_5O$ | O | $CH_2C{\equiv}CH$ | 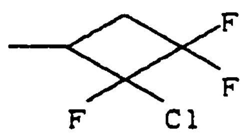 |
| $C_2H_5O$ | O | $CH_2C{\equiv}CH$ | 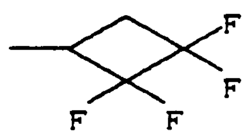 |
| $C_2H_5O$ | S | $CH_2C{\equiv}CH$ | 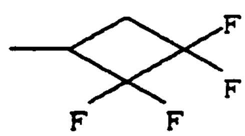 |
| $C_2H_5O$ | S | $CH_2C{\equiv}CH$ | 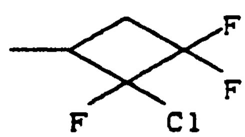 |

Verwendet man bei der Herstellungsvariante a) beispielsweise Thiophosphorsäurechlorid-O-ethylester-S-propylester und 2,3,3-Trifluor-2-chlor-cyclobutanol-1 als Ausgangsstoffe, so kann der entsprechende Reaktionsablauf durch das folgende Formelschema dargestellt werden:

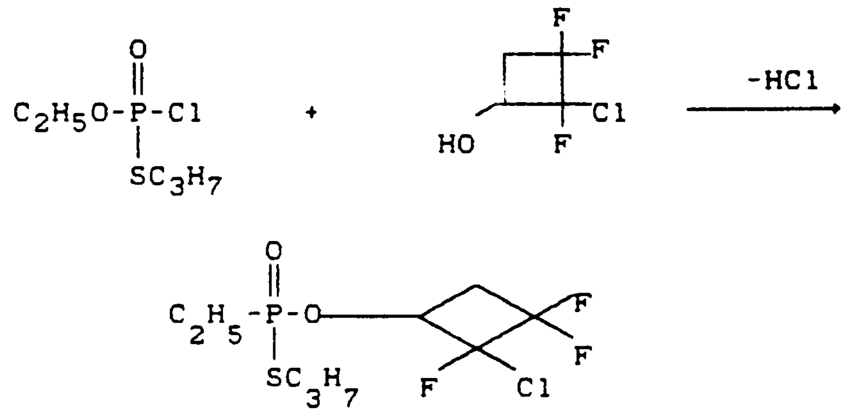


Verwendet man bei der Herstellungsvariante b) beispielsweise Thiophosphorsäure-S-butylester-dichlorid, Methanol und 2,2,3,3-Tetrafluorbutanol-1, so kann die Reaktion durch das folgende Formelschema dargestellt werden:

1. Schritt:

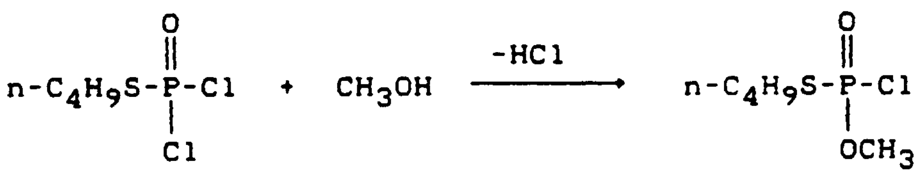


2. Schritt

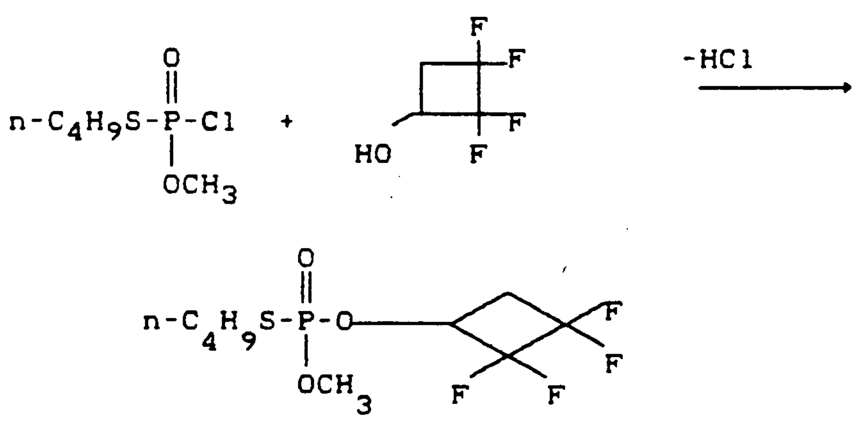


Die als Ausgangsstoffe für die Verfahrensvarianten (a), (b) und (c) zu verwendenden Phosphor(n)säure-Derivate der allgemeinen Formeln (II), (IV) und (VI) sind bekannt oder können nach bekannten Verfahren hergestellt werden (vgl. "Methoden der organischen Chemie" (Houben-Weyl) Bd. E2, 1982, Georg Thieme Verlag Stuttgart, New York, S. 300ff. und S. 487ff.).

Die Ausgangsstoffe der Formel (VII) sind neu und Teil der vorliegenden Erfindung. Sie werden gemäß dem ersten Reaktionsschritt der Verfahrensvarianten (c) erhalten. Ihre Isolierung und Reinigung erfolgt nach den üblichen Methoden, z. B. durch Destillation oder Chromatographie.

Die bei den erfindungsgemäßen Verfahren als Ausgangsstoffe verwendeten Cyclobutanol-Derivate sind durch die Formel (III) definiert.

Als Beispiele für die Verbindungen der Formel (III) seien genannt:
3,3,2-Trifluor-2-chlor-cyclobutanol-1; 3,3-Difluorcyclobutanol-1; 3,3,2-Trifluor-2-chlor-1-methyl-cyclobutanol-1 und deren Natrium-, Kalium- und Lithiumsalze.

Unter den Verbindungen der Formel (III) sind die Verbindungen der Formel (IIIa)

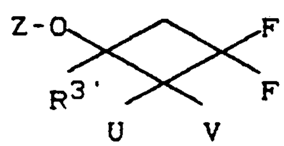

(IIIa)

neu, wobei in Formel (IIIa)

$R^{3'}$ für Wasserstoff oder Alkyl (vorzugsweise Wasserstoff steht,
U für Wasserstoff, Fluor oder Chlor (vorzugsweise Chlor steht,
V für Wasserstoff, Fluor oder Chlor (vorzugsweise Fluor) steht, und
Z für Wasserstoff oder ein Äquivalent eines Alkalimetallions steht,

mit der Maßgabe, daß U und V nicht beide für Fluor stehen.

Man erhält die Cyclobutanol-Derivate der Formel (IIIa), indem man Alkenylacetate der Formel (XI),

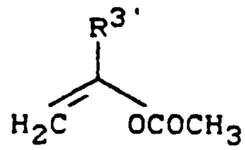

(XI)

in welcher

$R^{3'}$ die bei Formel (IIIa) genannte Bedeutung hat, mit halogensubstituierten Alkenen der Formel (XII)

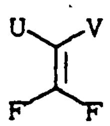

(XII)

in welcher

U und V die bei Formel (IIIa) genannte Bedeutung haben, gegebenenfalls in Gegenwart von Lösungsmitteln und gegebenenfalls in Gegenwart von Hydrochinon und/oder Dipenten umsetzt und in einem zweiten Reaktionsschritt die Acetylgruppe mit z.B. Hydrazin abspaltet (s. auch W.H Sharkey in Fluorine Chem. Rev. Vol. 2, S. 1, 1968, M. Decker, New York und Herstellungsvorschriften).

Die übrigen Verbindungen der Formel (III) können entsprechend oder nach bekannten Methoden und Verfahren erhalten werden.

Die für die Endprodukte der Formel (I) bzw. (Ia) aufgeführten bevorzugten Definitionen und Bereiche gelten für die jeweiligen Ausgangsstoffe in entsprechender Weise.

Als Verdünnungsmittel für die erfindungsgemäße Verfahrensvariante (a) kommen praktisch alle inerten organischen Verdünnungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon sowie Dimethylsulfoxid, Tetramethylensulfon und Hexamethylphosphorsäuretriamid.

Zur Durchführung der erfindungsgemäßen Verfahrensvarianten (a) setzt man auf 1 Mol Phosphor-Derivat der Formel (II) 1 bis 2 Mol, vorzugsweise 1,0 bis 1,8 Mol Cyclobutanol der Formel (III) ein.

Die Umsetzung wird im allgemeinen in einem geeigneten Verdünnungsmittel durchgeführt. Die Aufarbeitung geschieht nach üblichen Methoden. Die neuen Verbindungen fallen zum Teil in Form von Ölen an, die sich zum teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Als Verdünnungsmittel für die erfindungsgemäßen Verfahrensvarianten (b) und (c) können praktisch alle inerten organischen Verdünnungsmittel verwendet werden. Vorzugsweise werden die Verdünnungsmittel verwendet, die im Zusammenhang mit der Beschreibung der erfindungsgemäßen Verfahrensvarianten (a) genannt wurden.

Die Verfahrensvarianten (a), (b) und (c) können gegebenenfalls in Gegenwart von Basen durchgeführt werden. Als Basen können alle üblichen Basen Verwendung finden. Besonders bewährt haben sich Alkalicarbonate und -alkoholate, wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Tetraethylendiamin (DABCO) und Pyridin.

Die erfindungsgemäßen Verfahrensvarianten (a), (b) und (c) werden im allgemeinen bei Temperaturen zwischen -70 °C und +110 °C durchgeführt. Bevorzugt wird der Bereich zwischen -40 °C und 80 °C.

Die Umsetzungen werden im allgemeinen bei Normaldruck durchgeführt.

Zur Durchführung der erfindungsgemäßen Verfahrensvariante (b) setzt man auf 1 Mol der Verbindung der Formel (IV) 1 bis 1,6 Mol, vorzugsweise 1 bis 1,4 Mol des Alkohol-Derivates der Formel (V) und 1 bis 2, vorzugsweise 1 bis 1,8 Mol des Cyclobutanolderivates der Formel (III) ein.

Zur Durchführung der erfindungsgemäßen Verfahrensvariante (c) setzt man auf ein Mol der Verbindung der Formel (VI) 1 bis 1,6 Mol, vorzugsweise 1 bis 1,4 Mol, des Cyclobutanol-Derivates der Formel (III), 1 bis 2, vorzugsweise 1 bis 1,8 Mol, der Verbindungen der Formel (VIIIa) bzw. (VIIIb) und 1 bis 2, vorzugsweise 1 bis 1,8 Mol, der Alkylhalogenide der Formel (X) ein.

Die Umsetzung wird im allgemeinen in einem geeigneten Verdünnungsmittel und gegebenenfalls in Gegenwart eines Säureakzeptors durchgeführt. Die Aufarbeitung geschieht nach üblichen Methoden.

Die erfindungsgemäßen Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia

spp., Earias insulana, Heliothis spp., Spodoptera exigua, Mamestra brassicae, Panolis flammea, Prodenia litura, Spodoptera spp., Trlchoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp,, Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich durch eine hohe insektizide, akarizide und vor allem nematizide Wirksamkeit aus. Sie lassen sich insbesondere gegen pflanzenschädigende Insekten, wie beispielsweise gegen die Raupen der Kohlschabe (Plutella maculipennis) oder gegen die Larven der Meerrettichblattkäfer (Phaedon cochleariae), sowie gegen pflanzenschädigende Milben, wie beispielsweise gegen die gemeine Spinnmilbe (Tetranychus urticae) einsetzen. Sie eignen sich daneben hervorragend zur Bekämpfung von Bodeninsekten und Nematoden und lassen sich beispielsweise zur Bekämpfung von Phorbia antiqua-Maden oder von Nematoden der Gattung Meloidogyne incognita einsetzen. Auch eine gute wurzelsystemische Wirkung, beispielsweise gegen Phaedon cochleariae-Larven ist hervorzuheben. Die nematizide Wirkung der erfindungsgemäßen Wirkstoffe läßt sich auch im in vitro-Test beispielsweise gegen endoparasitisch lebende Nematoden der Gattung Caenorhabditis elegans bestätigen.

Außerdem besitzen die erfindungsgemäßen Wirkstoffe der Formel (I) eine hohe Wirkung gegen Hygiene- und Vorratsschädlinge und lassen sich beispielsweise zur Bekämpfung der orientalischen Schabe (Blatta orientalis) oder zur Bekämpfung des gemeinen Kornkäfers (Sitophilus granarius) einsetzen. Darüber hinaus lassen sich die erfindungsgemäßen Wirkstoffe mit besonders gutem Erfolg zur Bekämpfung von parasitisch lebenden Warmblüterschädlingen, (sowie Ekto- als auch Endoparasiten), wie beispielsweise gegen die Larven der Goldfliege (Lucilia cuprina), gegen Rinderzecken (Boophilus microplus), gegen Räudemilben (Psoroptes ovis) gegen Stechfliegen (Stomoxys calcitrans) oder gegen die Weideviehfliege (Musca autumnalis) einsetzen.

Daneben besitzen die erfindungsgemäßen Wirkstoffe der Formel (I) auch eine gute fungizide Wirksmkeit und lassen sich zur Bekämpfung von Pflanzenkrankheiten wie beispielsweise gegen den Erreger der Reisfleckenkrankheit (Pyricularia oryzae) oder gegen Schorf- und Botrytis-Erreger einsetzen.

In entsprechenden Aufwandmengen zeigen die erfindungsgemäßen Wirkstoffe der Formel (I) darüber hinaus eine herbizide Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen

Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff
und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus der handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken u.s.w. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer u.s.w. erreicht werden können.

Die Anwendung der erfindungsgemäßen Wirktoffe geschieht auf diesen Gebiet in bekannter Weise, wie durch äußerliche Anwendung in Form beispielsweise des Tauchens (Dippen), Sprühens (Sprayen), Aufgießens (pour-on and spot-on) und des Einpuderns.

Vorzugsweise werden die erfindungsgemäßen Wirkstoffe als Pflanzenschutzmittel, insbesondere zur Bekämpfung von Insekten und Nematoden, eingesetzt.

Die Herstellung der erfindungsgemäßen Verbindungen soll anhand der folgenden Beispiele erläutert werden.

Herstellungsbeispiele

Beispiel 1

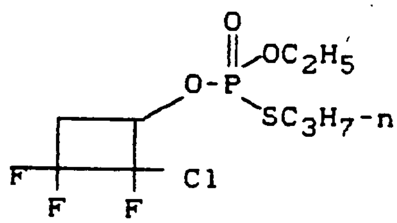

2,5 g (0,015 Mol) 3,3,2-Trifluor-2-chlor-cyclobutanol-1 werden in 50 ml Tetrahydrofuran gelöst und bei -40 °C mit 6 ml einer 23 %igen n-Butyllithium-Lösung (in Hexan) versetzt. Man rührt 0,5 h bei 20 °C und gibt dann 3,1 g (0,015 Mol) Thiophosphorsäure-S-propylester-O-ethylester-chlorid zur gut gerührten Reaktionsmischung. Man rührt 1 Tag bei 20 °C und gießt dann auf 200 ml Eis. Anschließend wird mit Methylenchlorid extrahiert, getrocknet ($MgSO_4$) und das Lösungsmittel abdestilliert. Dann löst man in dem Laufmittel Hexan:Aceton = 7:3 auf und filtriert über Kieselgel. Nach dem Abdestillieren des Lösungsmittels erhält man als Rückstand Thiophosphorsäure-S-propyl-O-ethyl-O-(3,3,2-trifluor-2-chlorcyclobutyl)triester.
Ausbeute: 2,6 g (53 % d.Th.)
$n_D^{20}$ = 1,4455

Beispiel 2

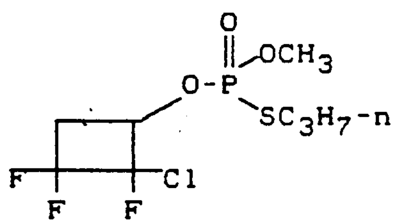

10 g (0,05 Mol) Thiophosphorsäure-S-propylester-dichlorid werden in 100 ml Toluol gelöst und bei -5° mit einer Mischung von 1,8 g (0,055 Mol) Methanol und 8,5 ml (0,06 Mol) Triethylamin und 10 ml Toluol versetzt. Man rührt eine Stunde bei 0 °C und fügt dann 2 g Triethylendiamin (DABCO) und 8,5 ml Triethylamin zur Reaktionsmischung. nach 10minütigem Rühren bei 0 °C werden langsam 10,3 g (0,062 Mol) 3,3,2-Trifluor-2-chlor-cyclobutanol-1, gelöst in 10 ml Toluol, zur Lösung getropft. Man rührt 1 h bei 20 °C, 1 Tag bei 50 °C und extrahiert dann die organische Phase mit Wasser. Nach Trocknen der Toluolphase ($MgSO_4$) wird das Lösungsmittel abdestilliert und mit Methylenchlorid als Laufmittel über Kieselgel filtriert. Man erhält 4,8 g eines Rohproduktes, das über eine Vigreux-Kolonnen destillativ gereinigt wird.
$Kp_{0,04}$ = 93 °C
$n_D^{20}$ = 1,4506
Ausbeute: 3,3 g (21 % der Theorie).

Beispiel 3

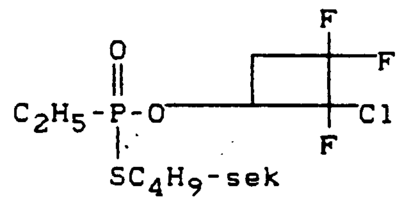

Beispiel 3a

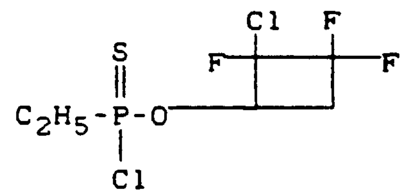

16,4 g (0,1 Mol) Ethanthiophosphonsäuredichlorid werden in 200 ml Tetrahydrofuran gelöst und bei 0 ° C mit 16,2 g (0,1 Mol) 2-Chlor-2,3,3-trifluorcyclobutanol versetzt. Anschließend tropft man langsam 13,5 g (0,1 Mol) Collidin zur gut gerührten Reaktionsmischung. Man rührt einen Tag bei 20 ° C, dann einen Tag bei 50 ° C und filtriert die abgekühlte Reaktionsmischung über Kieselgel (1 kg; Laufmittel : Methylenchlorid). Nach Destillation ($Kp_{0,03}$ = 42 ° C) erhält man 10,3 g (36 % der Theorie) Ethanthiophosphonsäure-(2-chlor-2,3,3-trifluorcyclobutyl)-esterchlorid.

Beispiel 3b

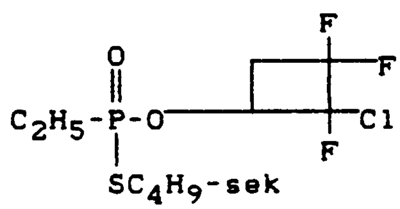

5,8 g (0,02 Mol) Ethanthiophosphonsäure-(2-chlor-2,3,3-trifluorcyclobutyl)-ester-chlorid werden in 80 ml Acetonitril gelöst und mit 2,5 g KOH-Pulver (0,045 Mol) versetzt. Man rührt 3 h bei 20 ° C, rotiert ein und versetzt den Rückstand mit 50 ml Diethylether. Anschließend fällt man das Kaliumsalz mit Hexan aus.

Diese Prozedur wird einmal wiederholt. Anschließend nimmt man den Rückstand wieder in 80 ml Acetonitril auf und versetzt die Reaktionsmischung mit 3,0 g (0,022 Mol) sek.-Butylbromid. Man erwärmt einen Tag auf 60 ° C und arbeitet dann mit Methylenchlorid/Wasser auf. Anschließend wird der nach Einrotieren der organischen Phase erhaltene Rückstand über Kieselgel chromatographiert (Laufmittel : Hexan : Aceton = 7 : 3).

Ausbeute: 1,2 g (18,5 % d. Th.)

$n_D^{20}$ = 1,4605.

Gemäß Beispiel 3a können die übrigen Verbindungen der Formel (VII) erhalten werden.

Verbindungen der Formel Ia

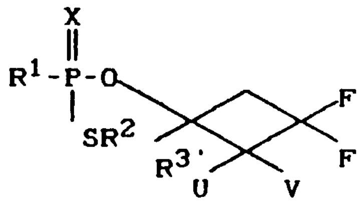

(Ia)

(X = O)

| Beispiel-Nr. | $R^1$ | $R^2$ | $R^{3'}$ | U | V | phys.Daten ($n_D^{20}$) |
|---|---|---|---|---|---|---|
| 4 | $C_2H_5O$ | sek-$C_4H_9$ | $CH_3$ | F | Cl | 1,4537 |
| 5 | $C_2H_5O$ | n-$C_3H_7$ | $CH_3$ | F | Cl | 1,4548 |
| 6 | $C_2H_5O$ | sek-$C_4H_9$ | H | F | Cl | 1,4467 |
| 7 | sek-$C_4H_9O$ | n-$C_3H_7$ | H | F | Cl | 1,4434 |
| 8 | i-$C_4H_9O$ | n-$C_3H_7$ | H | F | Cl | 1,4432 |
| 9 | $C_2H_5O$ | $CH_3$ | H | F | Cl | 1,4469 |
| 10 | $C_2H_5O$ | n-$C_3H_7$ | H | F | F | 1,4271 |
| 11 | $CH_3OCH_2CH_2O$ | n-$C_3H_7$ | H | F | Cl | 1,4395 |
| 12 | i-$C_3H_7O$ | n-$C_3H_7$ | H | F | Cl | 1,4445 |
| 13 | 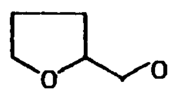 | n-$C_3H_7$ | H | F | Cl | 1,4600 |
| 14 | 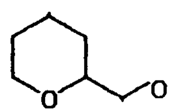 | n-$C_3H_7$ | H | F | Cl | 1,4672 |

(X = O)

| Beispiel-Nr. | $R^1$ | $R^2$ | $R^{3'}$ | U | V | phys.Daten ($n_D^{20}$) |
|---|---|---|---|---|---|---|
| 15 | $HC{\equiv}C-CH_2O$ | $n-C_3H_7$ | H | F | Cl | |
| 16 | $CH_2=CH-CH_2CH_2O$ | $n-C_3H_7$ | H | F | Cl | 1,4576 |
| 17 | $CH_2=CH-CH_2O$ | $n-C_3H_7$ | H | F | Cl | 1,4513 |
| 18 | $CH_3O$ | sek-$C_4H_9$ | H | F | Cl | |
| 19 | $C_2H_5O$ | 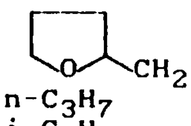 | H | F | Cl | 1,4680 |
| 20 | $OCH_2CH_2Cl$ | $n-C_3H_7$ | H | F | Cl | |
| 21 | $C_2H_5O$ | $i-C_3H_7$ | H | F | Cl | 1,4451 |
| 22 | $C_2H_5O$ | $n-C_3H_7$ | H | F | F | 1,4271 |
| 23 | 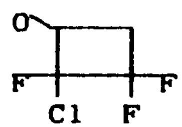 | $n-C_3H_7$ | H | F | Cl | 1,4464 |
| 24 | $C_2H_5$ | $n-C_3H_7$ | H | F | Cl | |
| 25 | $C_2H_5$ | $t-C_4H_9$ | H | F | Cl | |
| 26 | $CH_3$ | $n-C_3H_7$ | H | F | Cl | |
| 27 | $n-C_3H_7$ | $n-C_3H_7$ | H | F | Cl | |
| 28 | $C_2H_5$ | $n-C_3H_7$ | H | F | F | |
| 29 | $CH_3O$ | $n-C_3H_7$ | H | F | F | |
| 30 | $n-C_3H_7$ | $n-C_3H_7$ | H | F | F | |

(X = O)

| Beispiel-Nr. | $R^1$ | $R^2$ | $R^{3'}$ | U | V | phys.Daten ($n_D^{20}$) |
|---|---|---|---|---|---|---|
| 31 | $C_2H_5O$ | $CH_2C \equiv CH$ | H | F | F | |
| 32 | $C_2H_5O$ | $CHC \equiv CH$<br>$CH_3$ | H | F | F | |
| 33 | $C_2H_5O$ | $CH_2CH{=}CH_2$ | H | F | F | |
| 34 | $C_2H_5O$ | $CH_2CH(CH_3)_2$ | H | F | F | |
| 35 | $C_2H_5O$ | $C_2H_5$ | H | F | F | |
| 36 | $C_2H_5O$ | $CH_2{-}C \equiv CH$ | H | F | Cl | |
| 37 | $C_2H_5O$ | $CH(CH_3)C \equiv CH$ | H | F | Cl | |
| 38 | $C_2H_5O$ | $CH_2{-}CH{=}CH_2$ | H | F | Cl | |
| 39 | $C_2H_5O$ | $CH_2CH(CH_3)_2$ | H | F | Cl | |
| 40 | $C_2H_5O$ | $C_2H_5$ | H | F | Cl | |
| 41 | $C_2H_5O$ | $CH_2CH_2OCH_3$ | H | F | Cl | |
| 42 | $C_2H_5O$ | $CH_2CH_2OCH_3$ | H | F | F | |
| 43 | $C_2H_5O$ | $n{-}C_4H_9$ | H | F | F | |
| 44 | $C_2H_5O$ | $n{-}C_4H_9$ | H | F | Cl | |

(X = S)

| Beispiel-Nr. | $R^1$ | $R^2$ | $R^{3'}$ | U | V | phys.Daten ($n_D^{20}$) |
|---|---|---|---|---|---|---|
| 45 | $C_2H_5$ | n-$C_3H_7$ | H | F | Cl | |
| 46 | $C_2H_5$ | sek-$C_4H_9$ | H | F | Cl | 1,4954 |
| 47 | $C_2H_5$ | n-$C_3H_7$ | H | F | Cl | |
| 48 | $C_2H_5$ | t-$C_4H_9$ | H | F | Cl | 1,4960 |
| 49 | $CH_3$ | n-$C_3H_7$ | H | F | Cl | |
| 50 | $CH_3$ | n-$C_3H_7$ | H | F | F | |
| 51 | $C_2H_5$ | n-$C_3H_7$ | H | F | F | |
| 52 | $C_2H_5$ | sek-$C_4H_9$ | H | F | F | |
| 53 | n-$C_3H_7$ | n-$C_3H_7$ | H | F | F | |
| 54 | $CH_3O$ | n-$C_3H_7$ | H | F | Cl | |
| 55 | $CH_3O$ | n-$C_3H_7$ | H | F | F | |
| 56 | $C_2H_5O$ | n-$C_3H_7$ | H | F | F | 1,4721 |
| 57 | $C_2H_5O$ | t-$C_4H_9$ | H | F | Cl | |
| 58 | $C_2H_5O$ | sek-$C_4H_9$ | H | F | F | 1,4642 |
| 59 | $C_2H_5O$ | sek-$C_4H_9$ | H | F | Cl | 1,4825 |
| 60 | $C_2H_5O$ | n-$C_3H_7$ | H | F | Cl | 1,4825 |
| 61 | $C_2H_5O$ | n-$C_4H_9$ | H | F | Cl | |
| 62 | $C_2H_5O$ | n-$C_4H_9$ | H | F | F | |

(X = S)

| Beispiel-Nr. | $R^1$ | $R^2$ | $R^{3'}$ | U | V | phys.Daten ($n_D^{20}$) |
|---|---|---|---|---|---|---|
| 63 | $C_2H_5O$ | $CH_2-C\equiv CH$ | H | F | Cl | |
| 64 | $C_2H_5O$ | $CH(CH_3)C\equiv CH$ | H | F | Cl | |
| 65 | $C_2H_5O$ | $CH_2-CH=CH_2$ | H | F | Cl | |
| 66 | $C_2H_5O$ | $CH_2CH(CH_3)_2$ | H | F | Cl | |
| 67 | $C_2H_5O$ | $C_2H_5$ | H | F | Cl | |
| 68 | $C_2H_5O$ | $CH_2CH_2OCH_3$ | H | F | Cl | |
| 69 | $C_2H_5O$ | $CH_2CH_2OCH_3$ | H | F | F | |
| 70 | $C_2H_5O$ | $n-C_4H_9$ | H | F | F | |
| 71 | $C_2H_5O$ | $n-C_4H_9$ | H | F | Cl | |

Herstellung der Vorprodukte

Beispiel A1

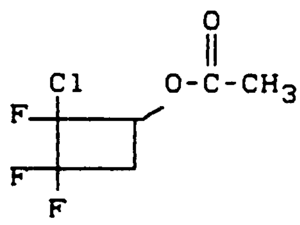

In einem 3 l Emaille-Autoklaven setzt man 1120 g (13 Mol) Vinylacetat mit 400 g (3,43 Mol) Trifluorchlorethylen in Gegenwart von 8 g Hydrochinon und 24 Tropfen Dipenten um. Die Reaktionstemperatur betrug 220 °C bei einer Reaktionsdauer von 3 Stunden. Das Reaktionsgemisch wird durch Destillation am Dünnschichter gereinigt.

| | |
|---|---|
| Ausbeute: | 526 g (75 % der Theorie) |
| $Kp_{44}$ mbar = | 73-85 °C |
| $n_D^{20}$ = | 1,3972 |
| $^1$H-NMR $\delta$ = | 2,1-3,1 ppm |
| | 3,9 ppm |
| | 4,2-4,7 ppm |

Beispiel A2

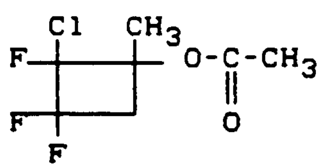

In einem 1,3 l Emaille-Autoklaven setzt man 500 g (5,0 Mol) Isopropylenacetat in Gegenwart von 4 g Hydrochinon und 10 Tropfen Dipenten mit 155 g (1,33 Mol) Trifluorchlorethylen 3 Stunden bei 220 °C um. Das Reaktionsgemisch wird durch Destillation gereinigt.

| | |
|---|---|
| Ausbeute: | 62 g (22 % der Theorie) |
| $Kp_{24}$ mbar = | 65 °C |
| $n_D^{20}$ = | 1,402 |
| $^1$H-NMR $\delta$ = | 1,8 ppm (3) |
| | 2,1 ppm (3) |
| | 2,7-3,1 ppm (2) |

Beispiel A3

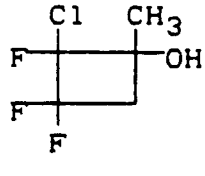

Zu 32 g (0,64 Mol) Hydrazinhydrat in 200 ml Diethylether addiert man bei 25 °C 62 g (0,27 Mol) Trifluorchlormethylcyclobutylacetat gemäß Beispiel A2 und rührt das Reaktionsgemisch 16 Stunden bei 20 °C nach. Die Reinigung erfolgt durch Destillation.

Ausbeute: 58 g
$Kp_{18}$ mbar = 50 °C
$n_D^{20}$ = 1,402
$^1$H-NMR δ = 1,5 ppm (3)
2,5-2,8 ppm (3)

Beispiel A4

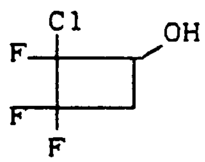

Zu 120 g (2,4 Mol) Hydrazinhydrat in 80,0 ml Diethylether addiert man bei 0 °C 450 g (2,2 Mol) Trifluorchlorcyclobutylacetat gemäß Beispiel A1. Man rührt das Reaktionsgemisch 14 Stunden bei 20 °C nach. Die Etherphase wird abgetrennt und über $Na_2SO_4$ getrocknet. Die Reinigung des Produkts erfolgt durch Destillation.

Ausbeute: 344 g (98 % der Theorie)
$Kp_{70}$ mbar = 70 °C
$n_D^{20}$ = 1,3965
$^1$H-NMR = 2,2-3,1 ppm (2)
3,8 ppm (1)
4,4 ppm (1)

Analog können die anderen Verbindungen der Formeln (III) bzw. (XII) hergestellt werden. 2,2,3,3-Tetrafluorcyclobutanol ist vorbeschrieben [W.D. Philipps, J. Chem. Phys. 25, 949 (1956)].

Die biologische Wirkung der erfindungsgemäßen Verbindungen soll anhand der folgenden Beispiele erläutert werden.

Beispiel A

Grenzkonzentrations-Test / Bodeninsekten

Testinsekt: Diabrotica balteata - Larven im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch Keine Rolle, entsprechend ist allein die Wirkstoffgewichtsmenge pro Volumeneinheit Boden, welche in ppm (mg/l) angegeben wird. Man füllt den Boden in 0,5 l Töpfe und läßt diese bei 20 °C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 % wenn noch genau so viele Testinsekten leben wie in der unbehandelten Kontrolle.

Bei diesem Test zeigten z.B. die Verbindungen der Herstellungsbeispiele 1, 4 und 10 bei einer beispielhaften Konzentration von 2,5 ppm einen Wirkungsgrad von 100 %.

Beispiel A1

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigten z.B. die Verbindungen der Herstellungsbeispiele 1, 2, 4, 5, 6, 10, 23 und 45 bei einer beispielhaften Wirkstoffkonzentration von 10 ppm einen Wirkungsgrad von 100 %.

Beispiel B

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z.B. die Verbindungen der Herstellungsbeispiele 1 und 10 bei einer beispielhaften Konzentration von 20 ppm eine Abtötungszahl von 100 %.

Beispiel C

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem

Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z.B. die Verbindungen der Herstellungsbeispiele 1 und 10 bei einer beispielhaften Konzentration von 20 ppm eine Abtötungszahl von 100 %.

Beispiel D

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27 °C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigten z.B. die Verbindungen der Herstellungsbeispiele 1, 4, 5, 6 und 10 bei einer beispielhaften Konzentration von 10 ppm einen Wirkungsgrad von 100 %.

Beispiel E

Grenzkonzentrations-Test

Testnematode: Globodera rostochiensis
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18 °C.

Nach sechs Wochen werden die Kartoffelwurzeln auf Zysten untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigten z.B. die Verbindungen der Herstellungsbeispiele 1, 4, 5, 6 und 10 bei einer beispielhaften Konzentration von 10 ppm einen Wirkungsgrad von 100 %.

Beispiel F

Aphis-Test (systemische Wirkung)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigte z.B. die Verbindung des Herstellungsbeispiels 1 bei einer beispielhaften Konzentration von 0,1 % nach 4 Tagen eine Abtötung von 100 %.

Beispiel G

$LT_{100}$-Test für Dipteren

Testtiere: Musca domestica, resistent
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist ($LT_{100}$).

Bei diesem Test zeigte z.B. die Verbindung des Herstellungsbeispiels 1 bei einer beispielhaften Konzentration von 0,1 % einen $LT_{100}$-Wert von 100 Minuten.

Beispiel H

$LD_{100}$-Test

Testtiere: Blattella germanica
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigte z.B. die Verbindung des Herstellungsbeispiels 1 bei einer beispielhaften Konzentration von 0,1 % eine Abtötung von 100 %.

**Patentansprüche**

1. O-(Halogencyclobutyl)-S-(alkyl)-(di)thiophosphon(r)-säureester der Formel (I)

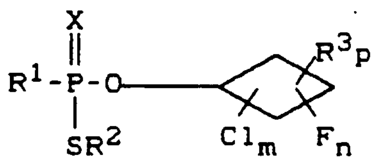

(I)

in welcher

X für Sauerstoff oder Schwefel steht,
p für null, eins oder zwei steht,
m für null, eins oder zwei steht,
n für 2, 3 oder 4 steht,
$R^1$ für Alkyl, für gegebenenfalls durch Sauerstoff oder Schwefel unterbrochenes Alkoxy, welches durch Halogen substituiert sein kann, für Alkenyloxy, Alkinyloxy, für Heterocyclylalkoxy oder für gegebenenfalls durch Alkyl und/oder Halogen substituiertes Cycloalkoxy steht,
$R^2$ für gegebenenfalls durch Sauerstoff oder Schwefel unterbrochenes Alkyl, welches durch Halogen substituiert sein kann, für Alkenyl, Alkinyl oder für Heterocyclylalkyl steht und
$R^3$ für Wasserstoff oder Alkyl steht.

2. O-Halogencyclobutyl-S-alkyl-(di)thiophosphon(r)-säureester gemäß Anspruch 1) der Formel (Ia)

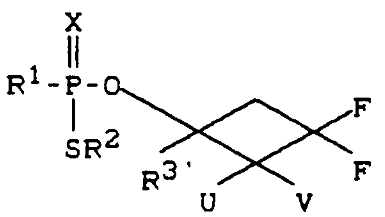

(Ia)

in welcher

$R^1$ für ($C_1$-$C_6$)-Alkyl, ($C_1$-$C_6$)-Alkoxy, ($C_2$-$C_6$)-Alkenyloxy, ($C_2$-$C_6$)-Alkinyloxy, (2-Hexahydropyranyl)methoxy, (2-Tetrahydrofuranyl)-methoxy, ($C_1$-$C_6$)-Alkoxy-($C_1$-$C_4$)-alkoxy, ($C_1$-$C_6$)-Alkylthio-($C_1$-$C_4$)-alkoxy oder 3,3,2-Trifluor-2-chlor-cyclobutoxy steht,
$R^2$ für ($C_1$-$C_6$)-Alkyl, ($C_2$-$C_6$)-Alkenyl, ($C_2$-$C_6$)-Alkinyl, (2-Hexahydropyranyl)-methyl, (2-Tetrahydrofuranyl)-methyl, ($C_1$-$C_6$)-Alkylthio-($C_1$-$C_4$)-alkyl oder ($C_1$-$C_6$)-Alkoxy-($C_1$-$C_4$-alkyl steht,
$R^{3'}$ für Wasserstoff oder ($C_1$-$C_4$)-Alkyl steht,
X für Sauerstoff oder Schwefel steht und
U für Wasserstoff, Fluor oder Chlor steht und
V für Wasserstoff, Fluor oder Chlor steht.

3. O-Halogencyclobutyl-S-alkyl-(di)thiophosphon(r)-säureester der Formel (Ia) gemäß Anspruch 2 in welcher

X für Sauerstoff oder Schwefel steht
$R^1$ für ($C_1$-$C_4$)-Alkyl, ($C_1$-$C_4$)-Alkoxy, ($C_2$-$C_4$)-Alkenyloxy, ($C_2$-$C_4$)-Alkinyloxy, (2-Tetrahydropyranyl)methoxy, ($C_1$-$C_4$)-Alkoxy-($C_1$-$C_4$)-alkoxy oder ($C_1$-$C_4$)-Alkylthio-($C_1$-$C_4$)alkoxy steht und
$R^2$ für ($C_1$-$C_4$)-Alkyl, ($C_2$-$C_4$)-Alkenyl, ($C_2$-$C_4$)-Alkinyl, (2-Tetrahydrofuranyl)-methyl, ($C_1$-$C_4$)-Alkoxy-($C_1$-$C_4$)-alkyl oder ($C_1$-$C_4$)-Alkylthio-($C_1$-$C_4$)-alkyl steht,
$R^{3'}$ für Wasserstoff oder Methyl steht,
X für Sauerstoff oder Schwefel steht,
U für Wasserstoff, Fluor oder Chlor steht und
V für Wasserstoff, Fluor oder Chlor steht.

**4.** Verbindungen der Formel

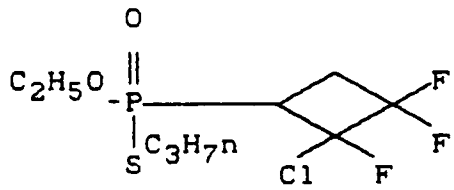

und

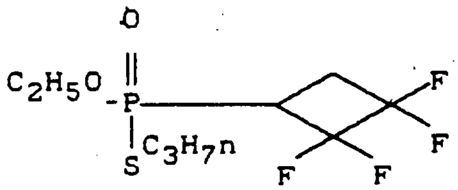

**5.** Verfahren zur Herstellung von O-Halogencyclobutyl-S-alkyl-(di)-thiophosphon(r)-säureestern der Formel (I)

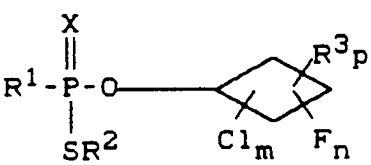

(I)

in welcher

- X für Sauerstoff oder Schwefel steht,
- p für null, eins oder zwei steht,
- m für null, eins oder zwei steht,
- n für 2, 3 oder 4 steht,
- $R^1$ für Alkyl, für gegebenenfalls durch Sauerstoff oder Schwefel unterbrochenes Alkoxy, welches durch Halogen substituiert sein kann, für Alkenyloxy, Alkinyloxy, für Heterocyclylalkoxy oder für gegebenenfalls durch Alkyl und/oder Halogen substituiertes Cycloalkoxy steht,
- $R^2$ für gegebenenfalls durch Sauerstoff oder Schwefel unterbrochenes Alkyl, welches durch Halogen substituiert sein kann, für Alkenyl, Alkinyl oder Heterocyclylalkyl steht und
- $R^3$ für Wasserstoff oder Alkyl steht,

dadurch gekennzeichnet, daß man

a) Thio- oder Dithiophosphor(n)säureester der Formel (II)

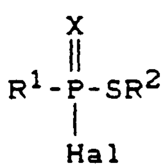

(II)

in welcher

- $R^1$, $R^2$ und X die oben angegebene Bedeutung haben und
- Hal für Halogen steht,

mit Cyclobutanol-Derivaten der Formel (III)

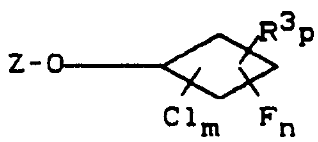 (III)

in welcher

$R^3$, n, m und p die oben angegebene Bedeutung haben und

Z für Wasserstoff oder ein Äquivalent eines Alkalimetallions steht,

gegebenenfalls in Gegenwart von Verdünnungsmitteln und gegebenenfalls in Gegenwart von Basen umsetzt,

oder daß man

b) zum Erhalt von Thio- oder Dithiophosphorsäureestern der Formel (I) in einem ersten Reaktionsschritt Säurehalogenide der Formel (IV)

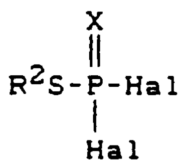 (IV)

in welcher

X, $R^2$ und Hal die oben angegebene Bedeutung besitzen, mit Verbindungen der Formel

$R^4$-H (V)

in welcher

$R^4$ für die bei $R^1$ aufgeführten Reste, außer Alkyl, steht,

gegebenenfalls in Gegenwart von Lösungsmitteln und gegebenenfalls in Gegenwart von Basen umsetzt und dann in einem anschließenden Reaktionsschritt mit Cyclobutanol-Derivaten der Formel (III)

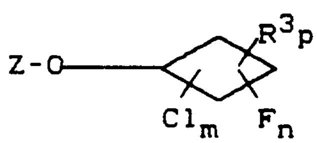 (III)

in welcher

Z, $R^3$, m, n und p die oben angegebene Bedeutung besitzen,

gegebenenfalls in Gegenwart von Verdünnungsmitteln und gegebenenfalls in Gegenwart von Basen umsetzt,

oder wenn man

c) zum Erhalt von Thio- oder Dithiophosphorsäureestern der Formel (I) in einem ersten Reaktionsschritt Säurehalogenide der Formel (VI)

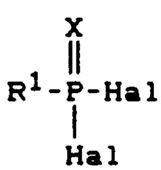 (VI)

in welcher

X, $R^1$ und Hal die oben angegebene Bedeutung haben,

mit Cyclobutanol-Derivaten der Formel (III)

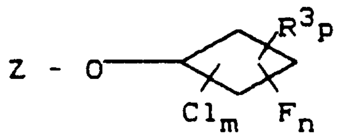

(III)

in welcher

Z, $R^3$, n, m und p die oben angegebene Bedeutung haben,

gegebenenfalls in Gegenwart von Verdünnungsmitteln und gegebenenfalls in Gegenwart von Basen umsetzt und in einem zweiten Reaktionsschritt die erhaltenen Verbindungen der Formel (VII)

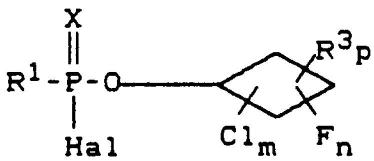

(VII)

in welcher

X, Hal, $R^1$, $R^3$, n, m und p die oben angegebene Bedeutung haben, mit Verbindungen der Formeln (VIIIa) bzw. (VIIIb)

$Z^1$-SH (VIIIa) bzw. $(Z^1)_2S$ (VIIIb)

in welcher

$Z^1$ für ein Äquivalent eines Alkalimetallions (vorzugsweise Natrium oder Kalium) steht,

gegebenenfalls in Gegenwart von Verdünnungsmitteln umsetzt und in einem dritten Reaktionsschritt die erhaltenen Verbindungen der Formel (IX)

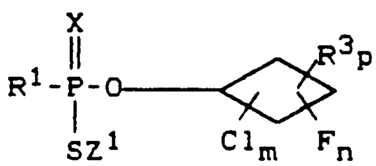

(IX)

in welcher

X, $Z^1$, $R^1$, $R^3$, m, n und p die oben angegebene Bedeutung haben, mit Alkylhalogeniden der Formel (X)

$R^2$ - Hal (X)

in welcher

$R^2$ und Hal die oben angegebene Bedeutung haben,

gegebenenfalls in Gegenwart von Verdünnungsmitteln umsetzt.

**6.** Schädlingsbekämpfungsmittel, gekennzeichnet durch einen Gehalt an mindestens einer Verbindung der Formel (I) gemäß Anspruch 1 oder 5.

**7.** Insektizide, akarizide und nematizide Mittel, gekennzeichnet durch einen Gehalt an mindesetns einer Verbindung der Formel (I) gemäß Anspruch 1 oder 5.

**8.** Verfahren zur Bekämpfung von Insekten und/oder Spinnentieren und/oder Nematoden, dadurch gekennzeichnet, daß man Verbindungen der Formel (I) gemäß Anspruch 1 oder 5 auf Insekten und/oder Spinnentiere und/oder Nematoden und/oder deren Lebensraum einwirken läßt.

**9.** Verwendung von Verbindungen der Formel (I) gemäß Anspruch 1 oder 5 zur Bekämpfung von Insekten und/oder Spinnentieren und/oder Nematoden.

**10.** Verfahren zur Herstellung von Schädlingsbekämpfungsmitteln, dadurch gekennzeichnet, daß man Verbindungen der Formel (I) gemäß Anspruch 1 oder 5 mit Streckmitteln und/oder oberflächenaktiven Mitteln vermischt.

**11.** Verbindungen der allgemeinen Formel (IIIa)

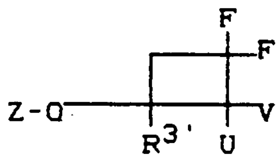

(IIIa)

in welcher

Z für Wasserstoff oder ein Äquivalent eines Alkalimetallions steht

$R^{3'}$ für Wasserstoff oder Alkyl steht,

U für Wasserstoff, Fluor oder Chlor steht und

V für Wasserstoff, Fluor oder Chlor steht,

mit der Maßgabe, daß U und V nicht beide für Fluor stehen.

**12.** Verbindungen der allgemeinen Formel (VII)

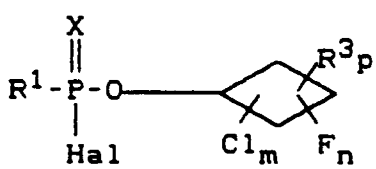

(VII)

in welcher

X für Sauerstoff oder Schwefel steht,

p für null, eins oder zwei steht,

m für null, eins oder zwei steht,

n für 2, 3 oder 4 steht,

$R^1$ für Alkyl, für gegebenenfalls durch Sauerstoff oder Schwefel unterbrochenes Alkoxy, welches durch Halogen substituiert sein kann, Alkenyloxy, Alkinyloxy, für Heterocyclylalkoxy oder für gegebenenfalls durch Alkyl und/oder Halogen substituiertes Cycloalkoxy steht,

$R^3$ für Wasserstoff oder Alkyl steht.

Hal für Halogen steht.

## Claims

**1.** O-Halogenocyclobutyl S-alkyl (di)thiophosph(on)ates of the formula (I)

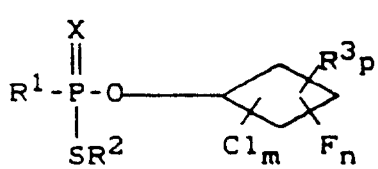

(I)

in which

X represents oxygen or sulphur,

p represents zero, one or two,

m represents zero, one or two,
n represents 2, 3 or 4,
$R^1$ represents alkyl, alkoxy which is optionally interrupted by oxygen or sulphur and can be substituted by halogen, or alkenyloxy, alkinyloxy, heterocycloalkoxy or cycloalkoxy which is optionally substituted by alkyl and/or halogen,
$R^2$ represents alkyl which is optionally interrupted by oxygen or sulphur and can be substituted by halogen, or alkenyl, alkinyl or heterocyclylalkyl and
$R^3$ represents hydrogen or alkyl.

**2.** O-Halogenocyclobutyl S-alkyl (di)thiophosph(on)ates according to Claim 1 of the formula (Ia)

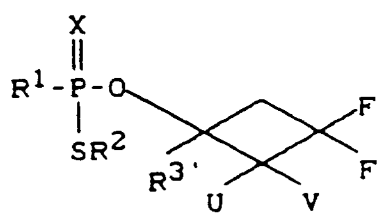

(Ia)

in which
$R^1$ represents ($C_1$-$C_6$)-alkyl, ($C_1$-$C_6$)-alkoxy, ($C_2$-$C_6$)-alkenyloxy, ($C_2$-$C_6$)-alkinyloxy, (2-hexahydropyranyl)methoxy, (2-tetrahydrofuranyl)methoxy, ($C_1$-$C_6$)-alkoxy-($C_1$-$C_4$)-alkoxy, ($C_1$-$C_6$)-alkoxy, ($C_1$-$C_6$)-alkylthio-($C_1$-$C_4$)-alkoxy or 3,3,2-trifluoro-2-chlorocyclobutoxy,
$R^2$ represents ($C_1$-$C_6$)-alkyl, ($C_2$-$C_6$)-alkenyl, ($C_2$-$C_6$)-alkinyl, (2-hexahydropyranyl)methyl, (2-tetrahydrofuranyl)methyl, ($C_1$-$C_6$)-alkylthia-($C_1$-$C_4$)-alkyl or ($C_1$-$C_6$)-alkoxy-($C_1$-$C_4$)-alkyl
$R^{3'}$ represents hydrogen or ($C_1$-$C_4$)-alkyl,
X represents oxygen or sulphur and
U represents hydrogen, fluorine or chlorine and
V represents hydrogen, fluorine or chlorine.

**3.** O-Halogenocyclobutyl S-alkyl (di)thiophosph(on)ates of the formula (Ia) according to Claim 2, in which
X represents oxygen or sulphur,
$R^1$ represents ($C_1$-$C_4$)-alkyl, ($C_1$-$C_4$)-alkoxy, ($C_2$-$C_4$)-alkenyloxy, ($C_2$-Cx4)-alkinyloxy, (2-tetrahydropyranyl)methoxy, ($C_1$-$C_4$)-alkoxy-($C_1$-$C_4$)-alkoxy or ($C_1$-$C_4$)-alkylthio-($C_1$-$C_4$)-alkoxy,
$R^2$ represents ($C_1$-$C_4$)-alkyl, ($C_2$-$C_4$)-alkenyl, ($C_2$-$C_4$)-alkinyl, (2-tetrahydrofuranyl)methyl, ($C_1$-$C_4$)-alkoxy-($C_1$-$C_4$)-alkyl or ($C_1$-$C_4$)-alkylthio($C_1$-$C_4$)-alkyl,
$R^{3'}$ represents hydrogen or methyl,
X represents oxygen or sulphur,
U represents hydrogen, fluorine or chlorine and
V represents hydrogen, fluorine or chlorine.

**4.** Compounds of the formula

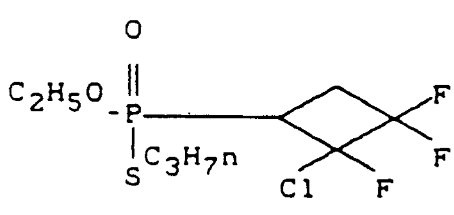

and

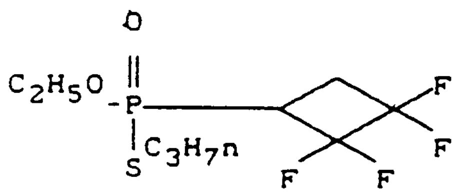

5. Process for the preparation of O-halogenocyclobutyl S-alkyl (di)thiophosph(on)ates of the formula (I)

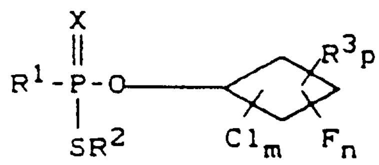

(I)

in which

X represents oxygen or sulphur,

p represents zero, one or two,

m represents zero, one or two,

n represents 2, 3 or 4,

$R^1$ represents alkyl, alkoxy which is optionally interrupted by oxygen or sulphur and can be substituted by halogen, or alkenyloxy, alkinyloxy, heterocyclyl-alkoxy or cycloalkoxy which is optionally substituted by alkyl and/or halogen,

$R^2$ represents alkyl which is optionally interrupted by oxygen or sulphur and can be substituted by halogen, or alkenyl, alkinyl or heterocyclyl-alkyl and

$R^3$ represents hydrogen or alkyl,

characterised in that

a) thiophosph(on)ates or dithiophosph(on)ates of the formula (II)

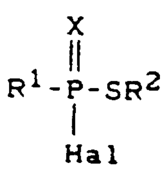

(II)

in which

$R^1$, $R^2$ and X have the meaning indicated above and

Hal represents halogen,

are reacted, if appropriate in the presence of diluents and if appropriate in the presence of bases, with cyclobutanol derivatives of the formula (III)

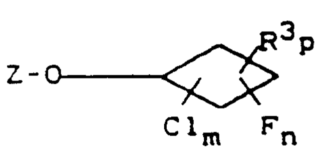

(III)

in which

$R^3$, n, m and p have the meaning indicated above and

Z represents hydrogen or an equivalent of an alkali metal ion,

or in that

b) thiophosphates or dithiophosphates of the formula (I) are obtained in a first reaction stage by reacting, if appropriate in the presence of solvents and if appropriate in the presence of bases, acid

halides of the formula (IV)

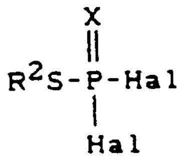

(IV)

in which

X, $R^2$ and Hal have the meaning indicated above, with compounds of the formula

$R^4$-H (V)

in which

$R^4$ represents the radicals listed under $R^1$, excepting alkyl,

and then reacting, in a subsequent reaction stage, if appropriate in the presence of diluents and if appropriate in the presence of bases, with cyclobutanol derivatives of the formula (III)

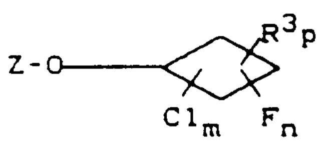

(III)

in which

Z, $R^3$, m, n and p have the meaning indicated above,

or in that

c) thiophosphates or dithiophosphates of the formula (I) are obtained in a first reaction stage by reacting acid halides of the formula (VI)

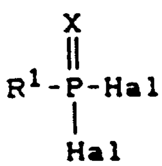

(VI)

in which

X, $R^1$ and Hal have the meaning indicated above,

with cyclobutanol derivatives of the formula (III)

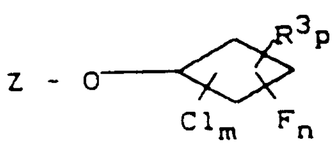

(III)

in which

Z, $R^3$, n, m and p have the meaning indicated above,

if appropriate in the presence of diluents and if appropriate in the presence of bases, and in a second reaction stage reacting the resulting compounds of the formula (VII)

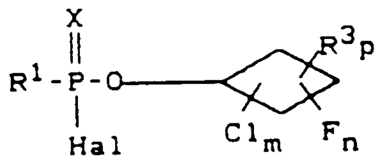

(VII)

in which

X, Hal, $R^1$, $R^3$, n, m and p have the meaning indicated above, with compounds of the formula (VIIIa) or (VIIIb)

$Z^1$-SH (VIIIa) or $(Z^1)_2S$ (VIIIb)

in which

$Z^1$ represents an equivalent of an alkali metal ion (preferably sodium or potassium),

if appropriate in the presence of diluents, and in a third reaction stage reacting the resulting compounds of the formula (IX)

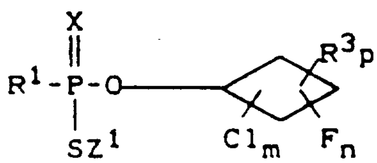

(IX)

in which

X, $Z^1$, $R^1$, $R^3$, m, n and p have the meaning indicated above, with alkyl halides of the formula (X)

$R^2$ - Hal (X)

in which

$R^2$ and Hal have the meaning indicated above,

if appropriate in the presence of diluents.

**6.** Pest-combating agent characterised in that it contains at least one compound of the formula (I) according to Claim 1 or 5.

**7.** Insecticidal, acaricidal and nematocidal agents characterised in that they contain at least one compound of the formula (I) according to Claim 1 or 5.

**8.** Process for combating insects and/or arachnids and/or nematodes, characterised in that compounds of the formula (I) according to Claim 1 or 5 are allowed to act on insects and/or arachnids and/or nematodes and/or the habitat thereof.

**9.** Use of compounds of the formula (I) according to Claim 1 or 5 for combating insects and/or arachnids and/or nematodes.

**10.** Process for the preparation of pest-combating agents, characterised in that compounds of the formula (I) according to Claim 1 or 5 are mixed with extenders and/or surface-active agents.

**11.** Compounds of the general formula (IIIa)

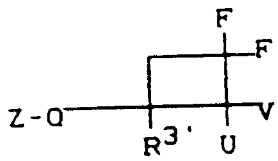

(IIIa)

in which

| | |
|---|---|
| Z | represents hydrogen or an equivalent of an alkali metal ion, |
| $R^{3'}$ | represents hydrogen or alkyl, |
| U | represents hydrogen, fluorine or chlorine and |
| V | represents hydrogen, fluorine or chlorine, |

subject to the proviso that U and V do not both represent fluorine.

**12.** Compounds of the general formula (VII)

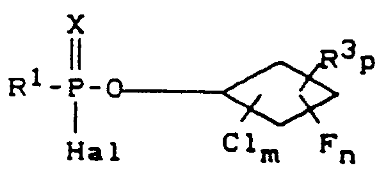

(VII)

in which

| | |
|---|---|
| X | represents oxygen or sulphur, |
| p | represents zero, one or two, |
| m | represents zero, one or two, |
| n | represents 2, 3 or 4, |
| $R^1$ | represents alkyl, alkoxy which is optionally interrupted by oxygen or sulphur and can be substituted by halogen, alkenyloxy, alkinyloxy, heterocyclylalkoxy or cycloalkoxy which is optionally substituted by alkyl and/or halogen, |
| $R^3$ | represents hydrogen or alkyl and |
| Hal | represents halogen. |

## Revendications

**1.** Esters O-(halogénocyclobutyl)-S-(alkyl)-(di)thio-phosphoniques(phosphoriques) de formule (I)

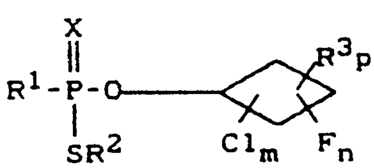

(I)

dans laquelle

| | |
|---|---|
| X | représente un atome d'oxygène ou un atome de soufre, |
| p | est égal à zéro, un ou deux, |
| m | est égal à zéro, un ou deux, |
| n | représente 2, 3 ou 4, |
| $R^1$ | représente un groupe alkyle; un groupe alcoxy éventuellement interrompu par un atome d'oxygène ou par un atome de soufre, qui peut être substitué par un atome d'halogène; un groupe alcényloxy, un groupe alcynyloxy; un groupe hétérocyclylalcoxy; ou encore un groupe cycloalcoxy éventuellement substitué par un groupe alkyle et/ou par un atome d'halogène, |

$R^2$ représente un groupe alkyle éventuellement interrompu par un atome d'oxygène ou par un atome de soufre, qui peut être substitué par un atome d'halogène; un groupe alcényle, un groupe alcynyle; ou encore un groupe hétérocyclylalkyle, et

$R^3$ représente un atome d'hydrogène ou un groupe alkyle.

**2.** Esters O-halogénocyclobutyl-S-alkyl-(di)thiophosphoniques(phosphoriques) selon la revendication 1 répondant à la formule (Ia)

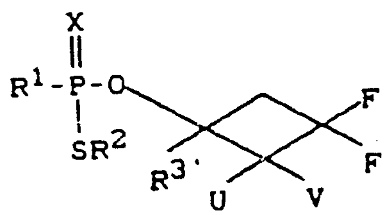

(Ia)

dans laquelle

$R^1$ représente un groupe alkyle en $C_1$-$C_6$, un groupe alcoxy en $C_1$-$C_6$, un groupe alcényl(en $C_2$-$C_6$)oxy, un groupe alcynyl(en $C_2$-$C_6$)oxy, un groupe (2-hexahydropyranyl)méthoxy, un groupe (2-tétrahydrofuranyl)méthoxy, un groupe alcoxy(en $C_1$-$C_6$)alcoxy en $C_1$-$C_4$, un groupe alkyl-(en $C_1$-$C_6$)thioalcoxy en $C_1$-$C_4$ ou encore un groupe 3,3,2-trifluoro-2-chlorocyclobutoxy,

$R^2$ représente un groupe alkyle en $C_1$-$C_6$, un groupe alcényle en $C_2$-$C_6$, un groupe alcynyle en $C_2$-$C_6$, un groupe (2-hexahydropyranyl)méthyle, un groupe (2-tétrahydrofuranyl)méthyle, un groupe alkyl(en $C_1$-$C_6$)thioalkyle en $C_1$-$C_4$ ou encore un groupe alcoxy(en $C_1$-$C_6$)alkyle en $C_1$-$C_4$,

$R^{3'}$ représente un atome d'hydrogène ou un groupe alkyle en $C_1$-$C_4$,

X représente un atome d'oxygène ou un atome de soufre,

U représente un atome d'hydrogène, un atome de fluor ou un atome de chlore, et

V représente un atome d'hydrogène, un atome de fluor ou un atome de chlore.

**3.** Esters O-halogénocyclobutyl-S-alkyl-(di)thiophosphoniques(phosphoriques) de formule (Ia) selon la revendication 2,

dans laquelle

X représente un atome d'oxygène ou un atome de soufre,

$R^1$ représente un groupe alkyle en $C_1$-$C_4$, un groupe alcoxy en $C_1$-$C_4$, un groupe alcényl(en $C_2$-$C_4$)oxy, un groupe alcynyl(en $C_2$-$C_4$)oxy, un groupe (2-tétrahydropyranyl)méthoxy, un groupe alcoxy(en $C_1$-$C_4$)alcoxy en $C_1$-$C_4$ ou un groupe alkyl(en $C_1$-$C_4$)thioalcoxy en $C_1$-$C_4$, et

$R^2$ représente un groupe alkyle en $C_1$-$C_4$, un groupe alcényle en $C_2$-$C_4$, un groupe alcynyle en $C_2$-$C_4$, un groupe (2-tétrahydrofuranyl)méthyle, un groupe alcoxy(en $C_1$-$C_4$)alkyle en $C_1$-$C_4$ ou un groupe alkyl(en $C_1$-$C_4$)thioalkyle en $C_1$-$C_4$,

$R^{3'}$ représente un atome d'hydrogène ou un groupe méthyle,

X représente un atome d'oxygène ou un atome de soufre,

U représente un atome d'hydrogène, un atome de fluor ou un atome de chlore, et

V représente un atome d'hydrogène, un atome de fluor ou un atome de chlore.

**4.** Composés de formule

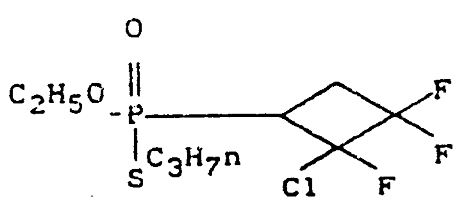

et

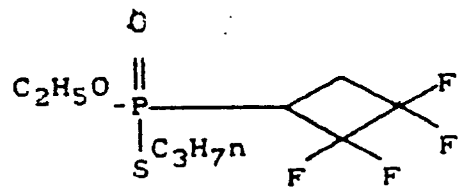

5. Procédé pour la préparation d'esters O-halogénocyclobutyl-S-alkyl-(di)thio-phosphoniques-(phosphoriques) de formule (I)

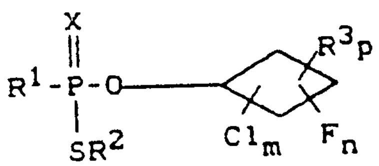

(I)

dans laquelle

X représente un atome d'oxygène ou un atome de soufre,

p est égal à zéro, un ou deux,

m est égal à zéro, un ou deux,

n représente 2, 3 ou 4,

$R^1$ représente un groupe alkyle; un groupe alcoxy éventuellement interrompu par un atome d'oxygène ou par un atome de soufre, qui peut être substitué par un atome d'halogène; un groupe alcényloxy, un groupe alcynyloxy; un groupe hétérocyclylalcoxy; ou encore un groupe cycloalcoxy éventuellement substitué par un groupe alkyle et/ou par un atome d'halogène,

$R^2$ représente un groupe alkyle éventuellement interrompu par un atome d'oxygène ou par un atome de soufre, qui peut être substitué par un atome d'halogène; un groupe alcényle, un groupe alcynyle; ou encore un groupe hétérocyclylalkyle, et

$R^3$ représente un atome d'hydrogène ou un groupe alkyle,

caractérisé en ce qu'on fait réagir

a) des esters thio- ou dithio-phosphoriques(phosphoniques) de formule (II)

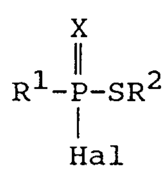

(II)

dans laquelle

$R^1$, $R^2$ et X ont la signification indiquée ci-dessus et

Hal représente un atome d'halogène,

avec des dérivés de cyclobutanol de formule (III)

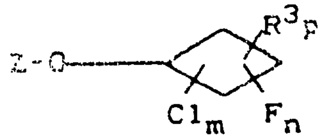

(III)

dans laquelle

$R^3$, n, m et p ont la signification indiquée ci-dessus et

Z représente un atome d'hydrogène ou un équivalent d'un ion de métal alcalin,

éventuellement en présence de diluants et éventuellement en présence de bases,

ou en ce que

b) pour obtenir les esters thio- ou dithiophosphoriques de formule (I), dans une première étape réactionnelle, on fait réagir des halogénures d'acides de formule (IV)

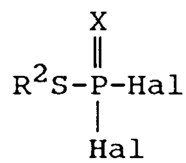

(IV)

dans laquelle

X, $R^2$ et Hal ont la signification indiquée ci-dessus, avec des composés de formule

$R^4$-H (V)

dans laquelle

$R^4$ représente les radicaux indiqués pour $R^1$, à l'exception d'un groupe alkyle,

éventuellement en présence de solvants et éventuellement en présence de bases, et ensuite dans une étape réactionnelle ultérieure, on les fait réagir avec des dérivés de cyclobutanol de formule (III)

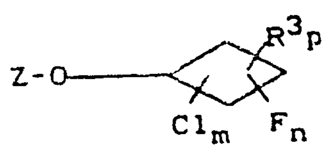

(III)

dans laquelle

Z, $R^3$, m, n et p ont la signification indiquée ci-dessus,

éventuellement en présence de diluants et éventuellement en présence de bases,

ou bien lorsque

c) pour obtenir les esters thio- ou dithiophosphoriques de formule (I), dans une première étape réactionnelle, on fait réagir des halogénures d'acides de formule (VI)

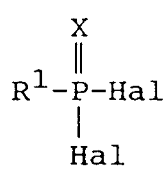

(VI)

dans laquelle

X, $R^1$ et Hal ont la signification indiquée ci-dessus,

avec des dérivés de cyclobutanol de formule (III)

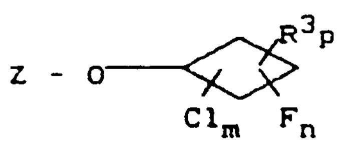

(III)

dans laquelle

Z, $R^3$, n, m et p ont la signification indiquée ci-dessus,

éventuellement en présence de diluants et éventuellement en présence de bases, et dans une deuxième étape réactionnelle, on fait réagir les composés obtenus de formule (VII)

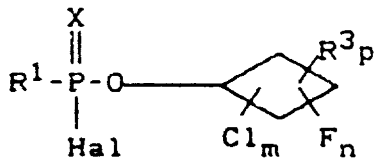

(VII)

dans laquelle

X, Hal, $R^1$, $R^3$, n, m et p ont la signification indiquée ci-dessus, avec des composés des formules (VIIIa) ou (VIIIb),

$Z^1$-SH (VIIIa) ou $(Z^1)_2S$ (VIIIb)

dans lesquelles

$Z^1$ représente un équivalent d'un ion de métal alcalin (de préférence de sodium ou de potassium),

éventuellement en présence de diluants, et dans une troisième étape réactionnelle, on fait réagir les composés obtenus de formule (IX)

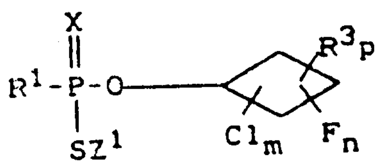

(IX)

dans laquelle

X, $Z^1$, $R^1$, $R^3$, m, n et p ont la signification indiquée ci-dessus, avec des halogénures d'alkyle de formule (X)

$R^2$-Hal (X)

dans laquelle

$R^2$ et Hal ont la signification indiquée ci-dessus,

éventuellement en présence de diluants.

**6.** Agents de lutte contre les parasites, caractérisés par une teneur en au moins un composé de formule (I) selon la revendication 1 ou 5.

**7.** Agents insecticides, acaricides et nématicides caractérisés par une teneur en au moins un composé de formule (I) selon la revendication 1 ou 5.

**8.** Procédé pour lutter contre des insectes et/ou des arachnides et/ou des nématodes, caractérisé en ce qu'on laisse agir des composés de formule (I) selon la revendication 1 ou 5, sur des insectes et/ou sur des arachnides et/ou sur des nématodes et/ou sur leur biotope.

**9.** Utilisation des composés de formule (I) selon la revendication 1 ou 5, pour lutter contre des insectes et/ou des arachnides et/ou des nématodes.

**10.** Procédé pour la préparation d'agents de lutte contre les parasites, caractérisé en ce qu'on mélange des composés de formule (I) selon la revendication 1 ou 5 avec des diluants et/ou des agents tensioactifs.

**11.** Composés répondant à la formule générale (IIIa)

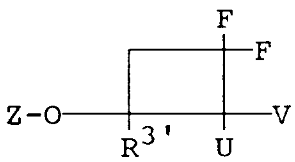

(IIIa)

dans laquelle

Z représente un atome d'hydrogène ou un équivalent d'un ion de métal alcalin,

$R^{3'}$ représente un atome d'hydrogène ou un groupe alkyle,

U représente un atome d'hydrogène, un atome de fluor ou un atome de chlore, et

V représente un atome d'hydrogène, un atome de fluor ou un atome de chlore,

avec cette mesure que U et V ne représentent pas, tous deux, un atome de fluor.

**12.** Composés répondant à la formule générale (VII)

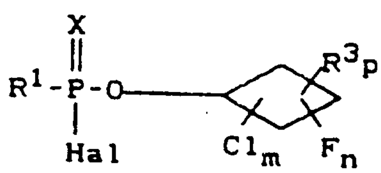

(VII)

dans laquelle

X représente un atome d'oxygène ou un atome de soufre,

p est égal à zéro, un ou deux,

m est égal à zéro, un ou deux,

n représente 2, 3 ou 4,

$R^1$ représente un groupe alkyle; un groupe alcoxy éventuellement interrompu par un atome d'oxygène ou par un atome de soufre, qui peut être substitué par un atome d'halogène; un groupe alcényloxy, un groupe alcynyloxy; un groupe hétérocyclylalcoxy; ou encore un groupe cycloalcoxy éventuellement substitué par un groupe alkyle et/ou par un atome d'halogène,

$R^3$ représente un atome d'hydrogène ou un groupe alkyle.

Hal représente un atome d'halogène.